# EUROPEAN PATENT APPLICATION

(11) **EP 3 563 755 A1**
(43) Date of publication of application: **06.11.2019**
(21) Application number: 18382301.2
(22) Date of filing: 30.04.2018
(51) Int. Cl.: A61B 3/10, A61B 3/16

(54) **SYSTEM AND METHOD FOR OBTAINING OCULAR TISSUE BIOMECHANICAL PARAMETERS**

(71) Applicant: Consejo Superior De Investigaciones Científicas (CSIC), 28006 Madrid (ES); Wellman Center for photomedicine, Cambridge, Massachusetts 02139 (US)
(72) Inventor: MARCOS CELESTINO, Susana, 28006 Madrid (ES); BIRKENFELD, Judith, 28006 Madrid (ES); YUN, Seok-Hyun, Belmont, MS 02478 (US)
(74) Representative: Balder IP Law, S.L.

(57) **Abstract**

The invention refers to a system (100) for obtaining biomechanical properties of ocular tissue, the system comprising:
- an air-puff generator (10) configured to generate a stepped-frequency train of air-puff pulses of different frequencies for delivery onto the ocular tissue;
- an imaging device (20) operatively coupled to the air-puff generator, the imaging device being configured to capture a plurality of axial positions of a region of the ocular tissue;
- processing means (30) configured to:
- process the plurality of axial positions provided by the imaging device for obtaining displacement and velocity of the region of the ocular tissue at the different frequencies; and to
- carry out frequency domain analysis of the obtained displacements and velocities of the region of the ocular tissue at the different frequencies to at least derive a mechanical resonance frequency of the ocular tissue.

The invention also relates to a method for obtaining biomechanical parameters of ocular tissue,

## Description

### TECHNICAL FIELD

The present invention generally relates to the field of ophthalmology. More specifically, the present invention relates to a non-contact technique of measuring biomechanical parameters of the cornea and intraocular measurements.

### STATE OF THE ART

The transparent part of the outer eye coat-the cornea-is the main refractive component of the eye. The inherent microstructure defines the material properties of the cornea, which in turn determine its shape. Deviations from the optimal aspheric corneal shape produce optical degradation of the retinal image leading to a decrease in the visual function. Therefore, measuring corneal biomechanical parameters is important for the characterization of the cornea in certain pathologies that compromise corneal structure (such as keratoconus), as well as prior to and following treatments, such as after refractive surgeries (LASer Insitu Keratomileusis, PhotoRefractive Keratectomy or Intrastromal Corneal Ring Segment implantation), UV collagen cross-linking treatment and keratoplasty. Measuring corneal biomechanical properties allows a better selection of the candidates for surgery, prediction of surgical outcomes and guiding of the surgical intervention, helps to improve the understanding of collagen cross-linking and could permit the early diagnosis of keratoconus.

A current limitation is that most methods for measuring corneal biomechanical properties operate on excised globes, corneas, flaps of corneal strips, and, are therefore only applicable *ex vivo*; very few systems are applicable *in vivo.*

Of the commercially available systems applicable *in vivo,* some analyse the geometrical corneal deformation following an air-puff, which procures a deformation in the cornea in the range of millimeters. These commercial systems, marketed as an ocular tonometers, provides corneal deformation parameters, which an indirectly related to mechanical and generally highly dependent on corneal thickness, IOP, the presence of the sclera, among others. Reconstruction of the inherent biomechanical properties from air-puff corneal deformation has been shown, although given the large deformation produced by the air-puff those are probed in a regime different to normal external pressure performed on the cornea, outside a linear range.

Ultrasound techniques are also applicable *in vivo* and estimate corneal elasticity by analysing the shear wave. But they are limited by a relatively low imaging resolution and the need of direct contact to the eye, which reduces patient comfort.

More recently, sound excitation has been used to induce vibrations in the eye globe. The advantage of sound is that it is applicable *in vivo* and a non-contact method. Also, sound-excitation is as simple as putting a loudspeaker in front of the sample. The disadvantage of sound is that the excitation is not localized to the cornea, which leads to bulk vibration, and it is generally difficult to control the excitation source which is highly dependent on the set-up.

Therefore, there is a need for a non-invasive approach for obtaining biomechanical parameters and intraocular pressure of the cornea that is robust, comfortable for the patient, independent of corneal geometry such as corneal thickness, and representative of the corneal response biomechanical response to standard pressure and treatments.

### DESCRIPTION OF THE INVENTION

A first aspect of the invention relates to a system for obtaining biomechanical properties of ocular tissue, the system comprising:
- an air-puff generator configured to generate a stepped-frequency train of air-puff pulses of different frequencies for delivery onto the ocular tissue;
- an imaging device operatively coupled to the air-puff generator, the imaging device being configured to capture a plurality of axial positions of a region of the ocular tissue;
- processing means configured to:
   - process the plurality of axial positions provided by the imaging device for obtaining displacement and velocity of the region of the ocular tissue at the different frequencies; and to
   - carry out frequency domain analysis of the obtained displacements and velocities of the region of the ocular tissue at the different frequencies to at least derive a mechanical resonance frequency of the ocular tissue.

Thus, the system of the present invention allows obtaining the mechanical resonance frequencies of the ocular tissue subjected to the air-puff pulses, which can be used to reconstruct biomechanical parameters of the ocular tissue and the eye's intraocular pressure (IOP). In comparison with existing air-puff-devices that work by deforming the cornea in a mm-range with a single air-puff excitation, the system of the present invention is configured to produce minimal deformation of the ocular tissue, in the µm range.

The ocular tissue is preferably the cornea, but it could also be the sclera.

In some embodiments, the processing means are further configured to carry out inverse calculation of biomechanical parameters of the ocular tissue and intraocular pressure using the displacement, velocity and mechanical resonance frequency, and in some embodiments along with a thickness of the ocular tissue, as inputs in a Fine-Element-Model based calculation.

It is also possible that the processing means are further configured to obtain biomechanical parameters of the ocular tissue from analytical models relating the vibrational frequency modes of membranes with intrinsic properties of the ocular tissue.

The imaging device is operatively coupled to the air-puff generator so that the air-puff train from the air-puff generator is synchronized with the imaging device.

In some embodiments, the imaging device is an imaging device having high motion sensitivity, with a displacement sensitivity in the range of micrometer, more preferably in the range of nanometers.

In some embodiments the imaging device is a phase-sensitive OCT, which is capable of measuring the displacement produced by the air-puff pulses onto the ocular tissue. In this case, the phase-sensitive and the OCT is synchronized and phase-locked with the air-puff generator; and the plurality of axial positions are a plurality of A-scans.

In some embodiments, the stepped-frequency train of air-puff pulses is linear, thereby providing the same number of axial positions per frequency analysed.

The different frequencies of the stepped-frequency train can range from 10 Hz to 600 Hz, in accordance with the expected resonance frequencies of ocular tissue; it would also be possible to sweep frequencies in the kHz, to detect resonance frequencies having higher harmonics. The frequency steps can be of 1 Hz, 10 Hz or 20 Hz; steps of 10 Hz are preferred so as not to make the measurement too long, or to miss a change in the resonance frequencies.

The air-puff generator is preferably configured to generate a stepped-frequency train of air-puff pulses of different frequencies for delivery onto the cornea, particularly at the cornea apex, or at the corneal limbus. In some embodiments, the air-puff generator comprises a nozzle, the nozzle being configured to point at a periphery of the cornea; this configuration prevents possible bulk movement along the z-axis.

In certain embodiments the air-puff generator comprises an air source, a valve connected to the processing means and a nozzle for delivering the train of air-puff pulses onto the ocular tissue, preferably having a duration of 1-10 ms. The air source can be pressurized between 100 kPa and 230 kPa, so as to produce a deformation in the ocular tissue within micrometer range. Keeping the corneal deformation within the µm range means working in the linear regime of the cornea or close to it, as larger deformations require the corneal material to be represented by a non-linear hyperelastic, and in addition, it is more consistent with day-to-day deformations the cornea may encounter, for example eye rubbing and blinking.

A further aspect of the invention refers to a method for obtaining biomechanical properties of ocular tissue, the method comprising:
- generating a stepped-frequency train of air-puff pulses of different frequencies for delivery onto the ocular tissue;
- capturing a plurality of axial positions of a region of the ocular tissue, the plurality of axial positions being operatively coupled to the stepped-frequency train of air-puff pulses of different frequencies;
- processing the plurality of axial positions to obtain displacement and velocity of the region of the ocular tissue at the different frequencies; and
- carrying out frequency domain analysis of the displacement and velocity of the region of the ocular tissue to at least derive a mechanical resonance frequency of the ocular tissue.

The stepped-frequency train of air-puff pulses of different frequencies can be generated by an air-puff generator. The plurality of axial positions of a region of the ocular tissue can be captured by an imaging device, which preferably has high motion sensitivity, the displacement sensitivity being in the range of micrometers.

In some embodiments, the step of processing the plurality of axial positions provided by the imaging device to obtain displacement and velocity of the region of the ocular tissue at the different frequencies comprises:
- applying Fourier transform to the plurality of axial positions from phase-time into space-time;
- calculating velocity of the ocular tissue using phase difference of the plurality of axial positions; and,
- applying Fourier transformation from space-time into space-frequency to obtain the displacement of the region of the ocular tissue at the different frequencies.

In some embodiments, the method further comprises obtaining biomechanical parameters of the ocular tissue by performing an inverse calculation of the biomechanical parameters of the ocular tissue and intraocular pressure using the displacement, velocity and mechanical resonance frequency, preferably along with thickness of the ocular tissue (preferably, the cornea) as inputs in a Fine-Element-Model based calculation.

In some embodiments, the method further comprises obtaining biomechanical parameters of the ocular tissue from analytical models relating the vibrational frequency modes of membranes with intrinsic properties of the ocular tissue.

The step of carrying out frequency-domain analysis of the obtained displacements and velocities preferably includes analysing the dependency of the amplitude of the moving ocular tissue with varying frequency. Instead of analysing deformation with time, the method of the present invention analyzes the displacement and velocity with frequency.

The mechanical resonance frequency can be used as a biomarker for biomechanical properties in the ocular tissue of an individual (preferably, with constant IOP). Also, the mechanical resonance frequency can be related to the intrinsic material properties, using analytical expressions of the vibration modes of a membrane. More generally, the mechanical resonance frequency, as well of displacement and velocity and ocular tissue geometry can be used as inputs to a Finite-Element Model based inverse optimization to reconstruct both the biomechanical parameters of the ocular tissue and the intraocular pressure IOP.

In some embodiments, the biomechanical parameters are elasticity or Young's modulus of the ocular tissue.

In another embodiment the system and method of the present invention is applicable to the sclera.

The different aspects and embodiments of the invention defined in the foregoing can be combined with one another, as long as they are compatible with each other.

Additional advantages and features of the invention will become apparent from the detail description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a schematic representation of the system of the invention according to a first possible embodiment.
Figure 2 shows the displacement and magnitude of (up/down) velocity of one *ex vivo* cornea during a frequency sweep between 20 Hz and 500-600 Hz.
Figure 3 shows the obtained mechanical resonance frequency for 9 different porcine corneas at a fixed IOP of 12 mmHg.
Figure 4 shows the displacement (µm) of one example cornea during a frequency sweep between 20 Hz and 500-600 Hz at four different IOP levels.
Figure 5 shows the schematics to derive corneal mechanical parameters and IOP from an inverse modeling optimization.

### DESCRIPTION OF PREFERRED EMBODIMENTS

The following description is not to be taken in a limiting sense but is given solely for the purpose of describing the broad principles of the invention. Next embodiments of the invention will be described by way of example, with reference to the above-mentioned drawings.

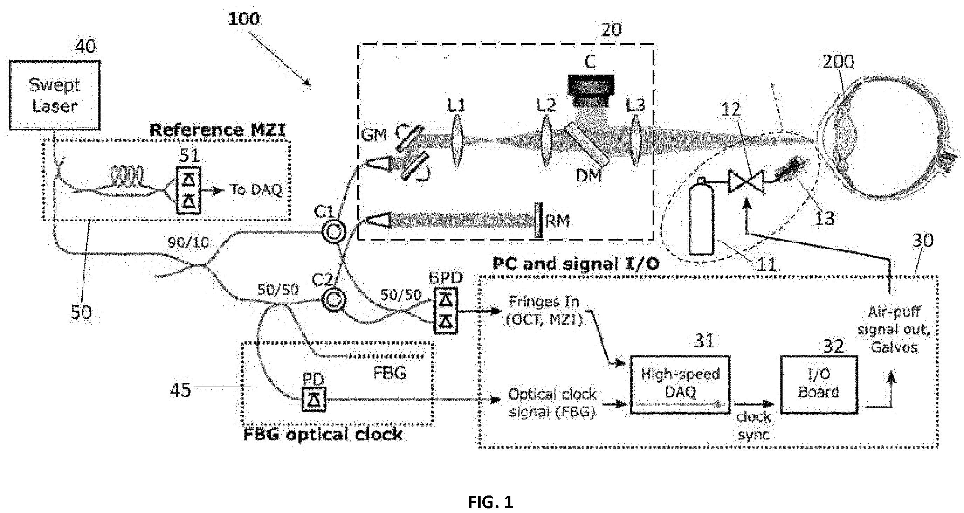

Figure 1 is a schematic representation of preferred embodiment of the system 100 for obtaining biomechanical parameters of ocular tissue 200, which is designed to send a train of air-puff pulses to the ocular surface to allow frequency domain analysis of the ocular mechanical resonances.

The main parts of the system of the invention are shown in this Figure 1 and are:
- a customizable air-puff generator 10;
- a custom build phase-sensitive swept source optical coherence tomograph, ssOCT 20; and
- a processor 30.

The air-puff generator 10 shown in this preferred embodiment comprises an air source 11, a high-speed solenoid valve 12 connected to the processor 30 and a needle nozzle 13. The air source 11 consists of a bottle of compressed air with an integrated gas pressure regulator to control the air-puff pressure. The high-speed solenoid valve 12 has a minimum switching time (on/off) of about 1 ms (it could be up to 10 ms), and a repetition accuracy of 0.1 ms. Its dimensions are 51x9x31 (LxWxH), and the weight is 44 g. The high-speed solenoid valve 12 works at 12-48 V DC and is connected to a DC power supply (not shown in this figure). The nozzle diameter of the needle nozzle 13 is 0.8 mm.

The air-puff train from the air-puff generator is synchronized and phase-locked with the OCT 20. The ssOCT 20 is used to obtain all measurements.

The ssOCT 20 comprises a camera C, several lenses L1, L2 and L3, a dichroic mirror DM and a galvanometric motor GM; and a sample arm 21 and a reference arm 22 which are connected to a swept source laser 40, using Fiber Bragg Grating (FBG) optical clock 45, and to a reference Mach-Zehnder Interferometer (MZI) 50. A balanced photodetector (BPD) 51 detects the reference MZI 50 and OCT signals, and the amplitude is normalized using the FBG optical clock circuit 45 and used to clock a DAQ 31 included as part of the processor 30. The synced signal is sent to an I/O board 32 of the processor 30, which in turn sends the synchronized signals to the high-speed solenoid valve 12. The swept-source laser 40 operates at 1310 nm. The ssOCT operates at a speed of 45000 A-scans/s, with an axial depth in air of 7.8 mm and an axial resolution was 15 µm; the displacement sensitivity is below 4 nm.

### Example 1

The following example illustrates an experimental procedure followed for assessing of ocular mechanical resonances using the system of the invention with the phase sensitive ssOCT 20 and the frequency-domain air-puff stimulation.

Freshly enucleated porcine eye globes were obtained, less than 24 h post-mortem. One eye was measured at a time. The eye was placed into a ring-like holder and carefully fixed with elastics. Care was taken that the cornea was not clamped after mounting. A medical needle was fed into the eyeball through the sclera. The needle connected with one end of a water conduit, the other end showed the water level, which allowed the reading and adjusting the intraocular pressure (IOP) of the eye globes. The IOP was set to 12 mmHg. The mounted eye was then aligned with the OCT beam so that the beam was positioned at the corneal apex. The air-puff nozzle was positioned about 3 mm from the sample, pointing onto the periphery of the cornea. This set-up was chosen to decrease the detection of translatory motion (bulk movement along the z-axis). The cornea was hydrated with saline solution throughout the measurement. The air pressure (at source) was set to 150 kPa.

A linear frequency sweep was applied with the puff-train from the air-puff generator. The frequencies were swept from 20 to 250 Hz; 20 to 50 0Hz; or 20 to 600 Hz, in steps of 10 or 20 Hz. Synchronized OCT M-mode imaging (A-scan at one location) was performed with the coupled ssOCT system during the frequency sweep. A typical OCT scan during a frequency sweep from 20-600 Hz in 10 Hz steps consisted of 265500 A-lines, whereby the number of A-lines for one frequency was 4500 (e.g. a scanning time of 5.9 s and 0.1 s, respectively).

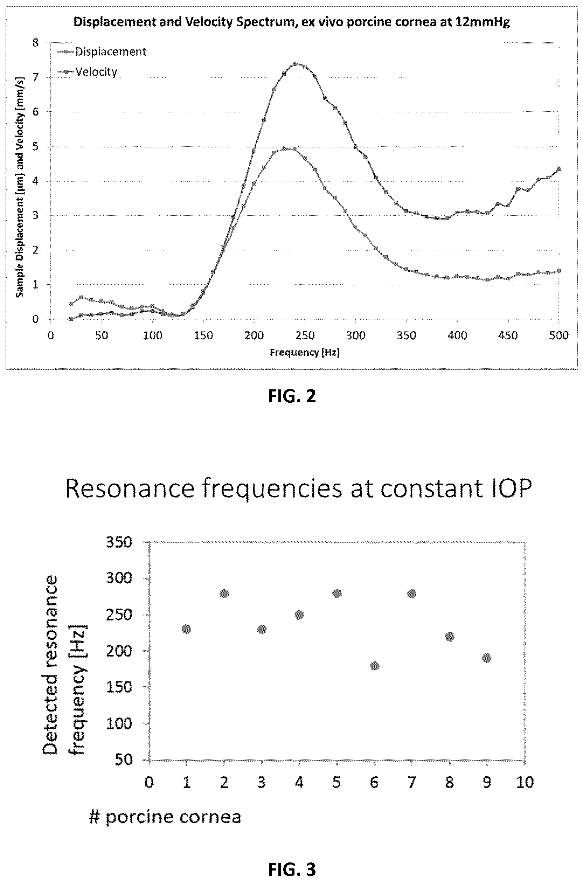

From the A-scan acquisition, the phase over time of the scanned corneal location was acquired. For data analysis, the raw fringes from OCT and reference MZI were phase-stabilized and a Fourier transformation was applied (k->z). The sample velocity was computed using the phase difference of the A-scans, and another Fourier transformation (t->f) was applied so that a function *U_{z}*(*z*,*t*) was obtained. With this function *U_{z}*(*z,t*), the sample displacement over time was obtained, as shown in Figure 2. From the frequency displacement curve, the mechanical resonance was determined as the frequency at the maximal amplitude.

This experiment was repeated for the other eight freshly enucleated porcine eyes (also at a constant IOP of 12 mmHg). A frequency-dependence of the amplitude and phase of corneal vibration was consistently observed for all corneas. Figure 3 shows the results for all measured corneas. The mean resonance frequencies over all samples was 230 ± 38 Hz.

### Example 2

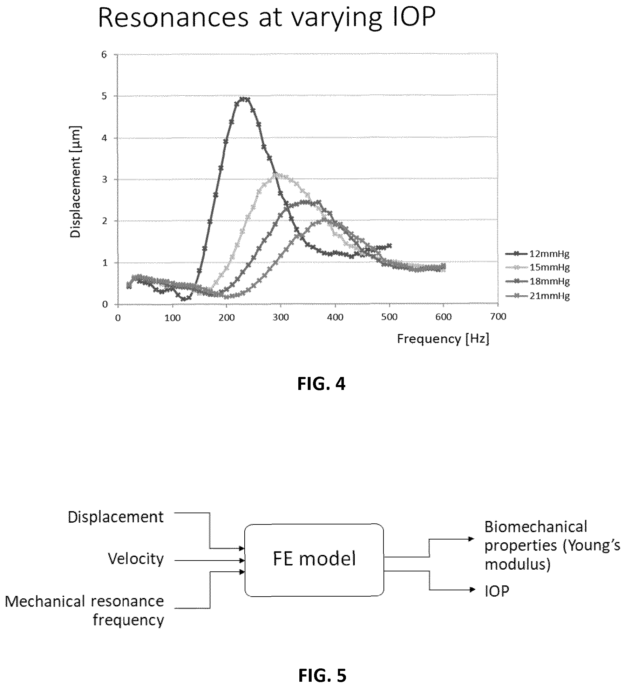

In another example, the ocular mechanical resonances were assessed for different lOPs. Five eye globes were used to obtain data at different IOP levels between 12 mmHg and 21 mmHg. All five eyes were measured at 12mm Hg; four of the five eye globes were additionally measured at IOP 15 and 21 mmHg, and three of the five eye globes were additionally measured at 18 mmHg. The experimental design consisted of mounting and imaging the cornea, as explained above. After every measurement, the IOP was increased in steps of 3 mmHg (12, 15, 18, 21 mmHg). Figure 4 shows the displacement-frequency curves of one cornea at the different lOPs. An increase in intraocular pressure was found to induce an increase in resonance frequency and a lower vibration amplitude. This was found in all investigated eyes.

### Example 3

Another experiment was carried out to assess mechanical resonances at different level of air puff pressure. One freshly enucleated eye globe was mounted at 12 mmHg IOP and the cornea was measured as described in Example 1. The air-puff pressure level at the air-source was varied from 50 kPa to 350 kPa (50, 100, 150 and 350 kPa) while the rest of the experimental set-up did not change. All measurements were done within five minutes. Table 1 shows the results for detected mechanical resonance frequencies and deformation amplitudes. The higher resonance frequency at 50 kPa might be due to the valve not working correctly at such low pressure level. Also, the displacement decreases significantly with low air pressure, so that maximum amplitude is harder to identify from the noise floor.

**Table 1: Resonance frequency for varying air-source pressure**

| Pressure level (at source) [kPa] | Resonance frequency [Hz] | Deformation amplitude [µm] |
|---|---|---|
| 50 | 240 | 1.3 |
| 100 | 210 | 2.7 |
| 150 | 210 | 4.1 |
| 350 | 200 | 5.8 |

### Example 4

In another set of experiments, the cornea was naturally dehydrated (no saline solution throughout all measurements) to investigate the cornea at different hydration levels, e.g. when the biomechanical parameters of the cornea changed over time. All measurements were performed as described in Example 1, but, as commented, the cornea was not rehydrated with saline solution during the measurements.

The resonance frequencies were investigated at 0 min (beginning of experiment), and after 90 min and 270 min. Table 2 shows the data obtain, which show an increase of resonance frequencies with progressive natural dehydration.

**Table 2: Mechanical resonance frequency of naturally dehydrating corneas.**

| | 0 min | 90 min | 270 min |
|---|---|---|---|
| Eye 1, difference in resonance frequency from 0 min [Hz] | 0 | | 65 |
| Eye 2, difference in resonance frequency from 0 min [Hz] | 0 | 30 | |
| Eye 3, difference in resonance frequency from 0 min [Hz] | 0 | 40 | |

In this preferred embodiment of the system of the present invention, the air-puff generator sends a stepped-frequency train of micron-level air-puffs onto the cornea to excite the cornea's mechanical resonances. The knowledge of driven resonance frequencies allows obtaining information about the cornea's biomechanical parameters and the eye's intraocular pressure (IOP). The resonance frequencies can be detected by a biomedical imaging device with high motion sensitivity, post processing the A-scans of the OCT and applying frequency domain analysis to the post-processed data from the OCT.

The system of the present invention is highly customizable and can be used in various set ups (puff excitation at corneal apex or at corneal limbus to induce resonance frequency).

Applications within the field of ophthalmology requiring quantification of corneal biomechanics include: biomarkers for corneal disease such as keratoconus, monitoring of treatments that modulate corneal biomechanics, predictions and surgical planning of corneal biomechanical response to corneal implants such as corneal inlays or intracorneal rings, corneal response to incisions, to corneal ablation. The present invention can be combined with other exploration methods to identify candidates for LASIK surgery and/or diagnosis of keratoconus.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. System (100) for obtaining biomechanical properties of ocular tissue, the system comprising:
- an air-puff generator (10) configured to generate a stepped-frequency train of air-puff pulses of different frequencies for delivery onto the ocular tissue;
- an imaging device (20) operatively coupled to the air-puff generator, the imaging device being configured to capture a plurality of axial positions of a region of the ocular tissue;
- processing means (30) configured to:
- process the plurality of axial positions provided by the imaging device for obtaining displacement and velocity of the region of the ocular tissue at the different frequencies; and to
- carry out frequency domain analysis of the obtained displacements and velocities of the region of the ocular tissue at the different frequencies to at least derive a mechanical resonance frequency of the ocular tissue.

2. The system of claim 1, wherein the imaging device is a phase-sensitive OCT, and the plurality of axial positions is a plurality of A-scans.

3. The system of any of claims 1-2, wherein the stepped-frequency train of air-puff pulses is linear.

4. The system of any of claims 1-3, wherein the different frequencies of the stepped-frequency train range from 20 Hz to 600 Hz, and in steps of 1 Hz, 10 Hz or 20 Hz.

5. The system of any of claims 1-4, wherein the air-puff generator comprises an air source (11), a valve (12) connected to the processing means (30) and a nozzle (13) for delivering the train of air-puff pulses onto the ocular tissue (200).

6. The system of any of claims 1-5, wherein the processing means (30) are further configured to carry out inverse calculation of biomechanical parameters of the ocular tissue and intraocular pressure using the displacement, velocity and mechanical resonance frequency as inputs in a Fine-Element-Model based calculation.

7. The system of any of claims 1-5, wherein the processing means (30) are further configured to obtain biomechanical parameters of the ocular tissue from analytical models relating the vibrational frequency modes of membranes with intrinsic properties of the ocular tissue.

8. Method for obtaining biomechanical parameters of ocular tissue (200), the method comprising:
- generating a stepped-frequency train of air-puff pulses of different frequencies for delivery onto the ocular tissue (200);
- capturing a plurality of axial positions of a region of the ocular tissue, the plurality of axial positions being operatively coupled to the stepped-frequency train of air-puff pulses of different frequencies;
- processing the plurality of axial positions to obtain displacement and velocity of the region of the ocular tissue at the different frequencies; and
- carrying out frequency domain analysis of the displacement and velocity of the region of the ocular tissue to at least derive a mechanical resonance frequency of the ocular tissue.

9. The method of claim 8, wherein the method further comprises obtaining biomechanical parameters of the ocular tissue (100) by performing an inverse calculation of the biomechanical parameters and intraocular pressure using the displacement, velocity and mechanical resonance frequency as inputs in a Fine-Element-Model based calculation, preferably also with thickness of the ocular tissue.

10. The method of claim 8, wherein the method further comprises obtaining biomechanical parameters of the ocular tissue from analytical models relating the vibrational frequency modes of membranes with intrinsic properties of the ocular tissue.

11. The method of any of claim 8-10, wherein the step of processing the plurality of A-scans provided by the imaging device to obtain displacement and velocity of the region of the ocular tissue (200) at the different frequencies comprises:
- applying Fourier transform to the plurality of axial positions from phase-time into space-time;
- calculating velocity of the ocular tissue (200) using phase difference of the plurality of axial positions; and,
- applying Fourier transformation from space-time into space-frequency to obtain the displacement of the region of the ocular tissue at the different frequencies.

12. The method of any of claims 8-11, which further comprises using the mechanical resonance frequency as a biomarker for biomechanical properties in the ocular tissue.

13. The method of any of claims 8-12, wherein the stepped-frequency train of air-puff pulses of different frequencies is generated by an air-puff generator (10).

14. The method of any of claims 8-12, wherein the plurality of axial positions of a region of the ocular tissue is captured by an imaging device (20).

15. The method of any of claims 8-14, wherein the method further comprises delivering the stepped-frequency train of air-puff pulses of different frequencies onto the cornea, preferably at the cornea apex, or at the corneal limbus.
